# EUROPEAN PATENT APPLICATION

(11) **EP 4 574 008 A1**
(43) Date of publication of application: **25.06.2025**
(21) Application number: 24196846.0
(22) Date of filing: 28.08.2024
(51) Int. Cl.: A61B 1/00, A61B 1/04, G02B 23/24

(54) **IMAGING APPARATUS FOR RIGID ENDOSCOPE, AND ENDOSCOPE SYSTEM**

(30) Priority: 22.12.2023 CN 202311784573
(71) Applicant: Wuhan Mindray Bio-Medical Scientific Co., Ltd., Wuhan City Hubei 430206 (CN); Shenzhen Mindray Bio-Medical Electronics Co., Ltd., Shenzhen, Guangdong 518057 (CN)
(72) Inventor: ZUO, Pengfei, Shenzhen, 518057 (CN); LI, Zhongqiang, Shenzhen, 518057 (CN); LV, Song, Wuhan, 430206 (CN); HUA, Weijie, Wuhan, 430206 (CN); SHI, Qiangyong, Shenzhen, 518057 (CN)
(74) Representative: KIPA AB

(57) **Abstract**

Provided are an imaging apparatus for a rigid endoscope and an endoscope system. The imaging apparatus includes a connection component, a key component, a handle component, and an optical component. The connection component includes a first connection part and a second connection part; the first connection part includes a first section and a second section. One end of the second section is connected with the first section, the other end of the second section is connected with the second connection part. The key component is arranged at the first connection part; the handle component is capable of being driven by an external force to move relative to the key component. In this disclosure, the key component is arranged at the first connection part, which is usually far away from a portion which is operated by a user, which improves a false triggering when the user operates the handle component.

## Description

### TECHNICAL FIELD

The disclosure relates to a technical field of medical device, and in particular, to an imaging apparatus for rigid endoscope, and an endoscope system.

### BACKGROUND

An imaging apparatus is a medical device which is used to observe lesion condition of tracheal tissue inside a patient. The imaging apparatus includes a handle component, an optical bayonet, and a key. The handle component is held by a user, the optical bayonet is connected with the handle component for being further connected with an external endoscope, and the key is triggered by the user. In related technologies, the key is usually arranged at the handle component of the imaging apparatus, which can easily cause accidental touch when the user operates the handle component, affecting usage of the user.

### SUMMARY

This disclosure at least aims to solve one of technical problems existing in the prior art. Therefore, this disclosure provides an imaging apparatus which is capable of improving the problem of false triggering when a user operates the handle component.

This disclosure also provides an endoscope system which uses an imaging apparatus.

According to a first embodiment of this disclosure, an imaging apparatus for a rigid endoscope is provided, which includes: a connection component, a key component, a handle component, and an optical component; wherein:
the key component is capable of being triggered by a user;
the handle component includes a housing and an image processing component; wherein the image processing component is accommodated inside an inner cavity of the housing, and is configured to receive an optical signal which is transmitted by the optical component, and generate an image signal;
the connection component includes a first connection part and a second connection part, wherein the second connection part is capable of being connected with the rigid endoscope, which connection is capable of being disconnected; the first connection part includes a first section and a second section, wherein the first section is connected with the handle component, the second section is connected with the second connection part; the first section is provided with a first channel and the second section is provided with a second channel, wherein the first channel and the second channel are joined together; the optical component includes a first optical element which is arranged inside the first channel, and a second optical element which is arranged inside the second channel; wherein an optical axis of the first optical element is substantially perpendicular to an optical axis of the second optical element;
wherein the key component is arranged at the first connection part, and electrically connected with the image processing component; the handle component is capable of being driven by an external force to move relative to the key component; the image processing component is further configured to receive the light signal, and generate the image signal, in response to a triggering of the key component.

The imaging apparatus for a rigid endoscope according to an embodiment of this disclosure has at least following beneficial effects.

In this disclosure, the key component is arranged at the first connection part, which is usually far away from a portion which is operated by a user, instead of being arranged at the handle component, which is capable of improving the problem of false triggering when the user operates the handle component.

In another embodiment of this disclosure, the imaging apparatus further includes a signal transmission component, which includes a first transmission part and a second transmission part; wherein the first transmission part is connected with the first connection part, and is further electrically connected with the key component; the second transmission part is connected with the handle component, and is further electrically connected with the image processing component; wherein the second transmission part is capable of rotating relative to the first transmission part, during a rotation of the handle component.

In another embodiment of this disclosure, the first transmission part includes a first conductor, the second transmission part includes a second conductor; wherein the first conductor and the second conductor are in contact with each other, and at least one of the first conductor and the second conductor is a circular structure.

In another embodiment of this disclosure, a signal, which is transmitted between the first transmission part and the second transmission part, is an electrical signal.

In another embodiment of this disclosure, a signal, which is transmitted between the first transmission part and the second transmission part, includes at least one of: a magnetic field signal, an optical signal, an electromagnetic wave signal, and a sound signal.

In another embodiment of this disclosure, the first transmission part includes an element for emitting magnetic field, the second transmission part includes an element for receiving magnetic field; the element for emitting magnetic field is configured to emit a magnetic field signal to the element for receiving magnetic field, in response to the triggering of the key component;
the first transmission part includes an element for emitting light, the second transmission part includes an element for receiving light; the element for emitting light is configured to emit an optical signal to the element for receiving light, in response to the triggering of the key component;
the first transmission part includes an element for emitting electromagnetic wave, the second transmission part includes an element for receiving electromagnetic wave; the element for emitting electromagnetic wave is configured to emit an electromagnetic wave signal to the element for receiving electromagnetic wave, in response to the triggering of the key component;
the first transmission part includes an element for emitting sound, the second transmission part includes an element for receiving sound; the element for emitting sound is configured to emit a sound signal to the element for receiving sound, in response to the triggering of the key component.

In another embodiment of this disclosure, the signal transmission component further includes a first flexible circuit board, wherein the first transmission part is further electrically connected with the key component through the first flexible circuit board; and/or
the signal transmission component further includes a second flexible circuit board, wherein the second transmission part is further electrically connected with the image processing component through the second flexible circuit board.

In another embodiment of this disclosure, one end of the second section of the first connection part is connected with the first section of the first connection part, the other end of the second section is connected with the second connection part; wherein a first end of the first section, which first end is away from the second section, is connected with the handle component; wherein, the key component is arranged at a second end of the first section, which second end is away from the handle component.

In another embodiment of this disclosure, the key component is arranged at a side of the first section, which side faces away the second section.

In another embodiment of this disclosure, the handle component is configured to be driven by the external force to rotate relative to the key component.

According to a second embodiment of this disclosure, an imaging apparatus for a rigid endoscope is provided, which includes: a connection component, a key component, a handle component, and an optical component; wherein:
the key component is capable of being triggered by a user;
the handle component is configured to be held by a user and/or used by a user to implement a manual adjustment;
the connection component includes a first connection part and a second connection part, wherein the second connection part is capable of being connected with the rigid endoscope, which connection is capable of being disconnected; the first connection part includes a first section and a second section; one end of the second section is connected with the first section, the other end of the second section is connected with the second connection part; a first end of the first section, which first end is away from the second section, is connected with the handle component; the first section is provided with a first channel and the second section is provided with a second channel, wherein the first channel and the second channel are joined together; the optical component includes a first optical element which is arranged inside the first channel, and a second optical element which is arranged inside the second channel; wherein an optical axis of the first optical element is substantially perpendicular to an optical axis of the second optical element;
wherein, the key component is arranged at a second end of the first section, which second end is away from the handle component.

In another embodiment of this disclosure, the one end of the second section is connected with the second end of the first section, so as to form an L-shaped structure.

In another embodiment of this disclosure, a central axis of the first channel is substantially perpendicular to a central axis of the second channel.

In another embodiment of this disclosure, the key component is arranged at a side of the second end, which side faces away the second section.

According to a third embodiment of this disclosure, an endoscope system is provided, which includes:
a light source host;
an imaging host;
a light guide beam, wherein one end of the light guide beam is connected with the light source host, the other end of the light guide beam is connected with a rigid endoscope, wherein the light guide beam is configured to provide a light source for the external endoscope; and
the imaging apparatus described in the previous embodiments, wherein the imaging apparatus is connected with the imaging host through a communication cable, so as to transmit an image signal which is obtained by the imaging apparatus to the imaging host.

Additional aspects and advantages of this disclosure are partially presented in the following description, some of which become apparent from the following description, or can be learned through practice of this disclosure.

### BRIEF DESCRIPTION OF THE DRAWINGS

Further explanations are made to this disclosure in combination with following attached drawings and embodiments.
FIG. 1 is a diagram of an endoscope system according to an embodiment of this disclosure.
FIG. 2 is a three-dimensional diagram of an imaging apparatus according to an embodiment of this disclosure.
FIG. 3 is a side view of the imaging apparatus shown in FIG. 2.
FIG. 4 is an exploded diagram of the imaging apparatus shown in FIG. 2.
FIG. 5 is a sectional view of the imaging apparatus shown in FIG. 2.
FIG. 6 is a sectional view, which shows connection relationships between a first connection part, partial housing, a signal transmission component, and an image processing component in the imaging apparatus shown in FIG. 2.
FIG. 7 is a three-dimensional diagram of a signal transmission component of the imaging apparatus shown in FIG. 2.

Reference number in the drawings:
Imaging apparatus 1000;
Connection component 100, first connection part 110, first section 111, first end 1111, second end 1112, second section 112, first channel 113, second channel 114, second connection part 120;
Key component 200;
Handle component 300, housing 310, upper housing 311, lower housing 312, flange 3121, image processing component 320;
Optical component 400, first optical element 410, second optical element 420, third optical element 430;
Signal transmission component 500, first transmission part 510, second transmission part 520, first flexible circuit board 530, second flexible circuit board 540;
Endoscope system 1, light source host 10, interface for a light guide beam 11, light guide beam 20, external endoscope 30, imaging apparatus 40, optical bayonet 41, imaging host 50, interface for an imaging head 51, display 60.

### DESCRIPTION OF THE PREFERRED EMBODIMENTS

Embodiments of this disclosure are described in detail below, and examples of the embodiments are shown in the accompanying drawings, where the same or similar reference numbers throughout represent the same or similar elements, or elements with the same or similar functions. The embodiments described below with reference to the accompanying drawings are exemplary and are only used to explain this disclosure, and should not be construed as limiting this disclosure.

In the description of this disclosure, it should be understood that an orientation or position relationship related to orientation description, such as up, down, front, back, left, right, etc., is based on an orientation or position relationship shown in the accompanying drawings, only for convenience of describing this disclosure and simplifying the description, and does not indicate or imply that the apparatus or element referred to must have a specific orientation, be constructed and operated in a specific orientation, and therefore cannot be understood as a limitation of this disclosure.

In the description of this disclosure, the meaning of "several" refers to one or more, and the meaning of "multiple" refers to two or more. The terms of "greater than one number", "less than one number", "more than one number", are understood as not including said number. While terms "above one number", "below one number", and "within one number", are understood as including said number. If described, "first" and "second" are terms which are only used to distinguish technical features, and cannot be understood as indicating or implying relative importance or implying a quantity or order of the indicated technical features.

In the description of this disclosure, unless otherwise explicitly limited, terms such as arrangement, installation and connection, should be broadly understood. Those skilled in the art can reasonably determine the specific meanings of the above terms in this disclosure based on the specific content of the technical solution.

In the description of this disclosure, the terms "one embodiment", "some embodiments", "an illustrative embodiment", "an example", "a specific example", or "some examples", refer to that specific feature(s), structure(s), material(s), or characteristic(s) described in conjunction with the embodiment(s) or example(s) is(are) included in at least one embodiment or example of this disclosure. In this description, the illustrative expressions of the above terms may not necessarily refer to same embodiment(s) or example(s). Moreover, the specific feature(s), structure(s), material(s), or characteristic(s) described can be combined in any one or more embodiments or examples in a suitable manner.

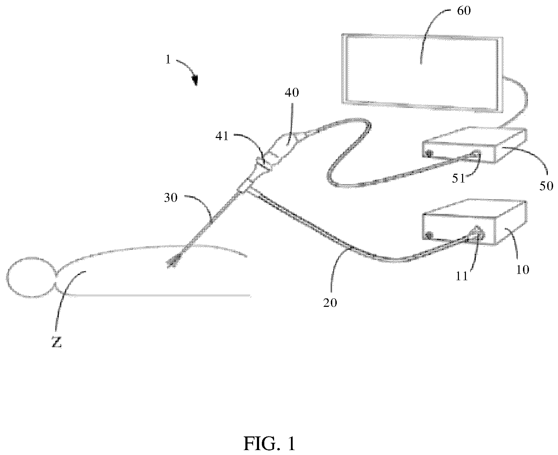

This disclosure discloses an imaging apparatus which is applied to a rigid endoscope, and this imaging apparatus is used in an endoscope system. For ease of understanding, a typical endoscope system is first described in conjunction with accompanying drawings. Referring to FIG. 1, an endoscope system 1 includes but is not limited to a light source host 10, a light guide beam 20, an external endoscope 30, an imaging apparatus 40, an optical bayonet 41, an imaging host 50, and a display 60.

One skilled in the art should understand that FIG. 1 is only an example of the endoscope system 1 and does not constitute a limitation on the endoscope system 1. The endoscope system 1 may include more or fewer components than shown in FIG. 1, or combine certain components, or include different components. For example, the endoscope system 1 may also include a dilator, a smoke control apparatus, an input/output apparatus, a network access apparatus, etc.

In this embodiment, one end of the light guide beam 20 is connected with an interface for a light guide beam 11 of the light source host 10, and the other end of the light guide beam 20 is connected with the external endoscope 30. The light guide beam 20 is configured to provide a light source for the external endoscope 30. One end of the imaging apparatus 40 is connected with an interface for an imaging head 51 of the imaging host 50 through a communication cable, so as to transmit an image signal which is obtained by the imaging apparatus 40 to the imaging host 50 for processing. The other end of the imaging apparatus 40 is connected with the external endoscope 30, so as to obtain an optical signal of the external endoscope 30.

The light source host 10 is configured to provide an illumination light source for a region Z to be observed. The illumination light source includes a visible illumination light source and a laser illumination light source which corresponds to a fluorescent reagent. The light source host 10 includes but is not limited to a laser light source, a visible light source, or a laser diode.

The light guide beam 20 is arranged inside a light output path of the light source host 10, and is connected with the external endoscope 30, so as to transmit the illumination light, which is generated by the light source host 10, to the region Z to be observed, through the light guide beam 20 and the external endoscope 30. A proximal end of the external endoscope 30 is connected with the imaging apparatus 40 through an optical bayonet 41.

At least one image sensor is arranged inside the imaging apparatus 40. The external endoscope 30 is configured to transmit the illumination light which is reflected by the region Z to be observed to at least one image sensor. Specifically, a distal end of the external endoscope 30 includes an insertion portion for being inserted into the region Z to be observed. The insertion portion is provided with a lens component. The external endoscope 30 is connected with at least one image sensor in the imaging apparatus 40 through the lens component, so that a scene light which is reflected by the region Z to be observed is projected onto at least one image sensor through the lens component.

At least one image sensor obtains image data in Bayer format. At least one image sensor is a Charge Coupled Device (CCD) sensor or a Complementary Metal Oxide Semiconductor (CMOS) sensor. In this embodiment, at least one image sensor is configured to alternately acquire light signals (such as a visible light image signal and a fluorescence image signal) which are reflected by the observed region in an interlaced manner, thereby improving image acquisition efficiency. Wherein the visible light image signal includes at least one of a blue-light image signal, a green -light image signal, and a red-light image signal.

The endoscope system 1 also includes an image processor. In some embodiments, the image processor is arranged inside the imaging host 50. In other embodiments, the image processor may also be provided within the imaging apparatus 40. In another embodiment, the external endoscope 30 and the imaging apparatus 40 may be an integrated electronic endoscope (such as a 3D electronic endoscope), with its image sensor located at a head end of the external endoscope 30. The image processor is configured to obtain the visible light image signal and the fluorescence image signal, and process the visible light image signal and the fluorescence image signal separately, so as to obtain a corresponding visible light image and a corresponding fluorescence image, as well as to fuse the visible light image and the fluorescence image for outputting a visible light image with a fluorescent marker. Display 60 is configured to display at least one of the visible light image, the fluorescent image, and the visible light image with the fluorescent marker.

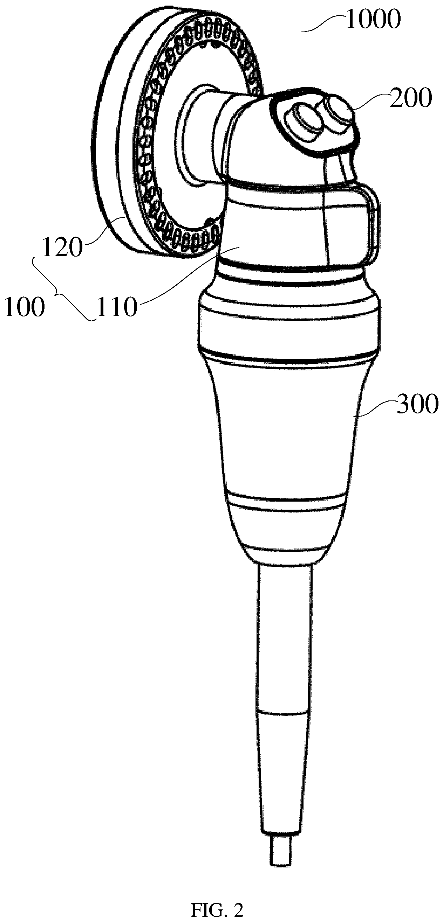

A first embodiment of this disclosure discloses an imaging apparatus 1000 for a rigid endoscope. Referring to FIG. 2, this embodiment differs from the imaging apparatus 40 in FIG. 1. For example, the imaging apparatus 40 in FIG. 1 is a linear imaging apparatus, and the light transmitted by an external endoscope can pass through a channel inside the imaging apparatus 40 along a straight line. However, the imaging apparatus 1000 in this embodiment has a generally L-shaped structure, and the light transmitted by the external endoscope needs to make a turn inside the imaging apparatus 1000. For the L-shaped imaging apparatus shown in FIG. 2, its typical structure is as follows. The L-shaped imaging apparatus includes a fixation portion and a handle portion. The fixation portion is connected with the external endoscope through, for example, the optical bayonet 41 in FIG. 1. The handle portion has an image sensor, such as one image sensor mentioned above. In actual application, the endoscope and the optical bayonet 41 rotate according to an operation of a user, which results in that a light signal which is transmitted from the endoscope to the image sensor cannot be received by the image sensor in a correct direction, and in turn results in that the image which is displayed on the display rotates to affect viewing of the user. Based on this, currently, the handle portion of the L-shaped imaging apparatus can rotate relative to the fixation portion, thereby driving the image sensor to rotate to adjust an orientation of the displayed image on the display, so as to enable the displayed image on the display to be in a correct orientation for viewing, thus making it easier for the user to view. In addition, the L-shaped imaging apparatus is also provided with a key which allows the user to control the imaging apparatus to perform shooting and other operations. Currently, the key is arranged at the handle portion of the L-shaped imaging apparatus. However, as mentioned earlier, the user needs to frequently operate the handle portion to rotate, which can easily trigger the key falsely and affect the operations of the user. Based on this, the imaging apparatus of this embodiment arranges the key on the fixation portion, which can significantly reduce the occurrence of false triggering of the key. The following is explained in conjunction with specific embodiments and accompanying drawings.

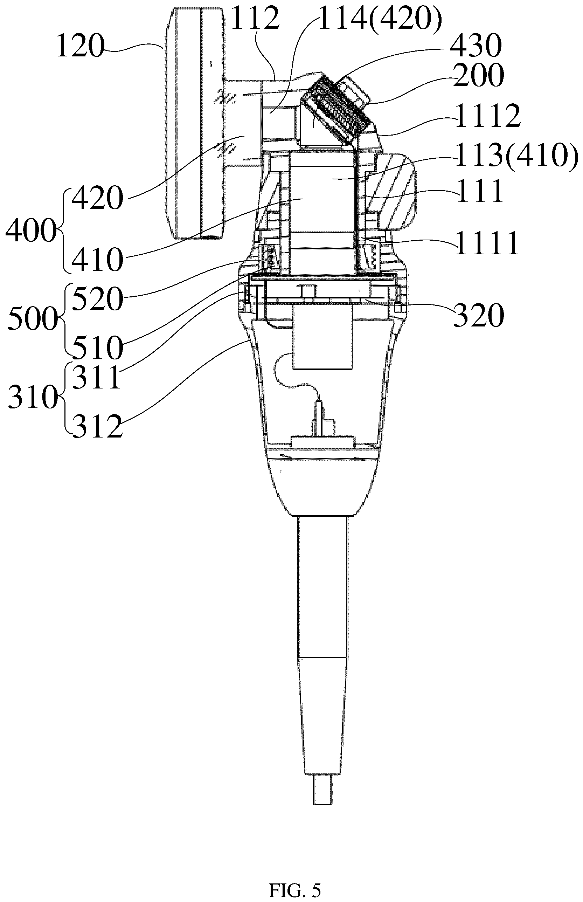

Referring to FIGS. 2 to 5, the imaging apparatus 1000 in this embodiment includes a connection component 100, a key component 200, a handle component 300, and an optical component 400 (shown in FIG. 5). The connection component 100 is configured to install an external endoscope, the handle component 300 is configured to be held by a user and/or used by a user to implement a manual adjustment; the key component 200 is capable of being triggered by the user, so as to control the imaging apparatus 1000 to perform an operation, and the optical component 400 is configured to form an optical path.

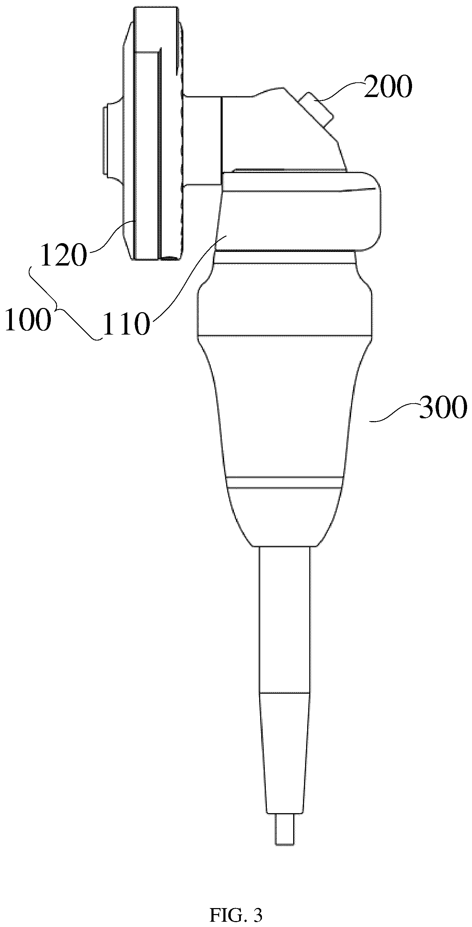

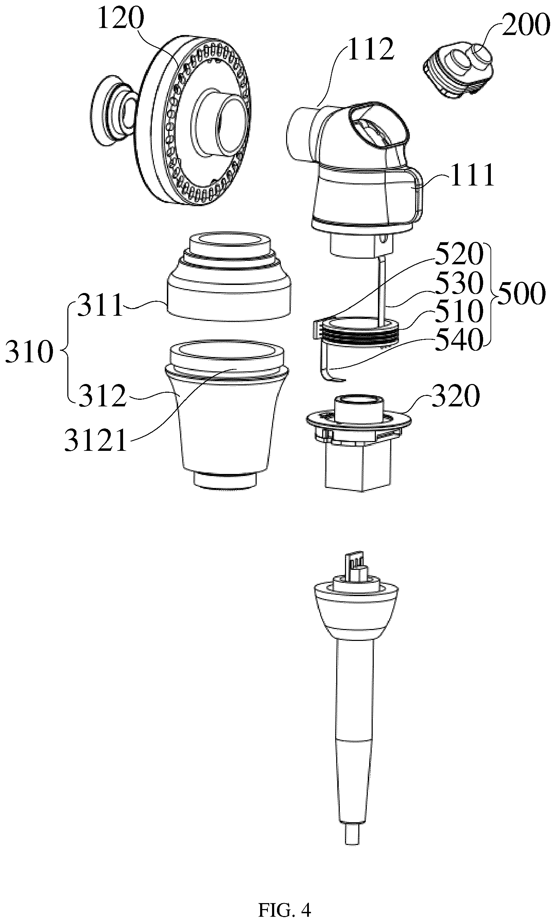

Referring to FIGS. 3 and 4, the connection component 100 includes a first connection part 110 and a second connection part 120 which are interconnected. The first connection part 110 is connected with the handle component 300, and the second connection part 120 is capable of being connected with an external endoscope, such as a rigid endoscope, which connection is capable of being disconnected. Specifically, the first connection part 110 includes a first section 111 and a second section 112 which are interconnected. For example, the first section 111 and the second section 112 are connected with each other as an integrated structure. The first section 111 is connected with the handle component 300, and the second section 112 is connected with the second connection part 120. In the illustrated embodiment, the first section 111 and the second section 112 are connected with each other through their respective ends. More specifically, the first section 111 is arranged along a vertical direction, and the second section 112 is arranged along a horizontal direction, so that the first section 111 and the second section 112 are substantially perpendicular to each other. It should be noted that the expressions "arranged substantially along a vertical direction", "arranged substantially along a horizontal direction", and "substantially perpendicular" mentioned here and in other parts of this disclosure are expressions that consider processing capacity. They include situations that "arranged along a vertical direction", "arranged along a horizontal direction", and "perpendicular", as well as situations in which a certain deviation exists comparing with a strict vertical state, a strict horizontal state and a strict perpendicular state.

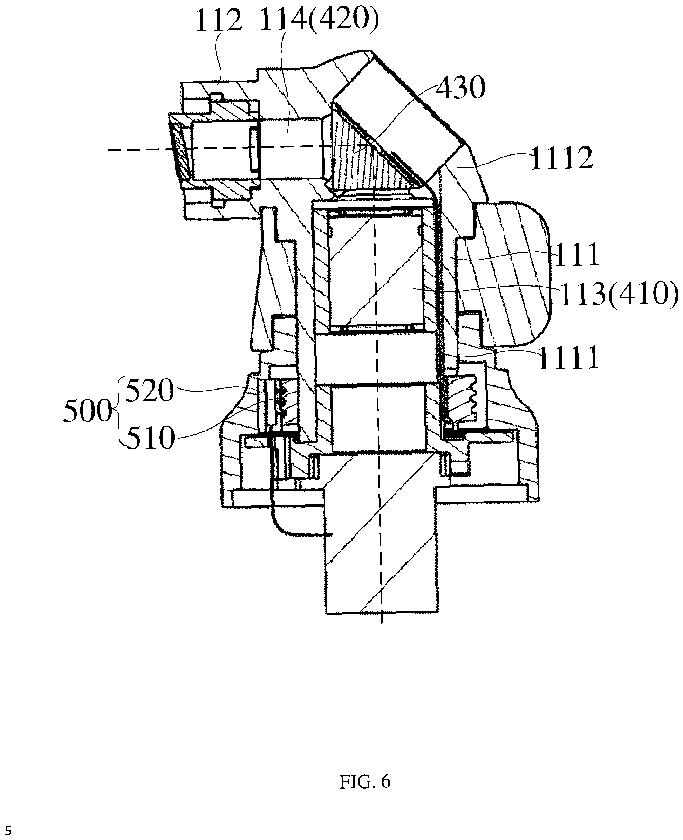

Referring to FIGS. 5 and 6, a dashed line in FIG. 6 represents an optical axis of the optical element. The first section 111 is provided inside with a first channel 113 and the second section 112 is provided inside with a second channel 114. The first channel 113 and the second channel 114 are joined with each other. Taking the example shown in the figure, the first channel 113 and the second channel 114 are joined through their respective ends. More specifically, the first channel 113 is arranged substantially along the vertical direction, and the second channel 114 is arranged substantially along the horizontal direction, so that the first channel 113 and the second channel 114 are substantially perpendicular to each other. The optical component 400 includes a first optical element 410 which is arranged inside the first channel 113, and a second optical element 420 which is arranged inside the second channel 114. An optical axis of the first optical element 410 is substantially perpendicular to an optical axis of the second optical element 420.

It should be noted that the optical component 400 is not limited to the first optical element 410 and the second optical element 420, as shown in FIGS. 5 and 6. The optical component 400 may also include a third optical element 430 which is arranged at a joint between the first channel 113 and the second channel 114. The optical signal, which is transmitted from the endoscope, passes through the first optical element 410 in a first direction (e.g. horizontal direction), changes its direction through the third optical element 430, and then passes through the second optical element 420 in a second direction (e.g. vertical direction), so as to be transmitted to the image sensor.

It should be noted that cross-sectional shapes of the first channel 113 and the second channel 114 are adjusted according to shapes of the optical elements. In the illustrated embodiment, both the first channel 113 and the second channel 114 are circular channels.

The handle component 300 includes a housing 310, which is connected with the first section 111 of the aforementioned first connection part 110, specifically connected with a lower end of the first section 111. In the embodiments shown in FIGS. 4 and 5, the housing 310 is in a separated structure, which includes an upper housing 311 and a lower housing 312, which are fixed to each other by welding, bonding, or other methods. For example, referring to FIG. 4, an upper end surface of the lower housing 312 is provided with a circle of flange 3121 which extends along an axial direction, and the upper housing 311 is sleeved at an outer side of the flange 3121. A lower end surface of the upper housing 311 is bonded or welded to the upper end surface of the lower housing 312. The housing 310 is arranged in a separated structure for easy installation of other internal components. In some further embodiments, a sealing structure is also provided between the upper housing 311 and the lower housing 312, for example, a sealing ring is provided between an inner wall surface of the upper housing 311 and an outer wall surface of the flange 3121.

The handle component 300 further includes an image processing component 320, which may include the aforementioned image sensor. The image processing component 320 is accommodated inside an inner cavity of the housing 310. In response to a triggering of the key component 200, the image processing component 320 receives an optical signal which is transmitted by the optical component 400 and generates an image signal. The image signal is transmitted to an external display to form an image. In this embodiment, the handle component 300 can be controlled by the user to move relative to the first connection part 110, such as rotating relative to the first connection part 110, thereby achieving a manual aligning function of the image.

The key component 200 is electrically connected with the image processing component 320, so as to enable the key component 200 to respond to a triggering of the user for controlling the image processing component 320. It should be noted that the key component 200 can be electrically connected with the image processing component 320 through a contact or non-contact manner, which is explained in subsequent embodiments. Referring to FIG. 2 to 5, the key component 200 is specifically arranged at the first connection part 110. When the handle component 300 is driven by the user, the key component 200 and the first connection part 110 can remain relatively stationary. In this embodiment, the key component 200 is arranged at the first connection part 110, which is usually far away from a portion which is operated by the user, instead of being arranged at the handle component 300, which is capable of improving the problem of false triggering when the user operates the handle component.

It should be noted that the key component 200 referred to in this disclosure includes a triggering part, which can be triggered by the user to generate a control signal. The specific triggering methods include but are not limited to pressing, touching, rotating, pushing, etc. Correspondingly, the triggering part can be a press-type triggering part, a touch-type triggering part, a rotation-type triggering part, and a push-type triggering part.

Based on the first embodiment, the imaging apparatus may further include other components, such as a focusing component. The focusing component includes an adjustment ring that is sleeved outside of the housing 310, such as outside the upper housing 311. The optical component 400 also includes an installation ring. The first optical element 410 is integrated into the installation ring. With the rotation of the adjustment ring, an optical module which is formed by the installation ring and the first optical element 410 can move along the axial direction of the housing 310, thereby achieving focusing. As a specific implementation of an axial movement of the optical module which is driven by the adjustment ring, an outer side of the installation ring has a driving shaft which extends radially along the housing 310, an inner side of the adjustment ring has an oblique groove, wherein the housing 310 is provided with a guide groove which is arranged along the axial direction. The driving shaft passes through the guide groove and is inserted into the oblique groove. In this way, as the adjustment ring rotates, the driving shaft slides inside the oblique groove on one hand and slides inside the guide groove on the other hand, which enables the optical module to move axially.

Based on the first embodiment, the imaging apparatus may also include other components, such as a signal transmission component 500. In this embodiment, the signal transmission component 500 is electrically connected with the key component 200 and the image processing component 320 respectively, so as to achieve a control of the image processing component 320 by the key component 200. In some embodiments, the signal transmission component 500 may be a wire or a flexible circuit board, and two ends of the wire or flexible circuit board are electrically connected with the key component 200 and the image processing component 320 respectively through plug-in terminals or soldering. In other embodiments, the signal transmission component 500 may also include multiple components. Specifically, referring to FIGS. 4 to 7, the signal transmission component 500 includes a first transmission part 510 and a second transmission part 520. The first transmission part 510 is connected with the first connection part 110 and is further electrically connected with the key component 200, and the second transmission part 520 is connected with the handle component 300 and is further electrically connected with the image processing component 320. When the user triggers the key component 200, the first transmission part 510 and the second transmission part 520 can transmit a signal. As mentioned earlier, in order to align the image, the handle component 300 frequently rotates during use. The signal transmission component 500 of this embodiment includes two independent parts, that is, the first transmission part 510 and the second transmission part 520. The first transmission part 510 is electrically connected with the key component 200, so as to maintain relatively stationary, the second transmission part 520 is electrically connected with the image processing component 320, so as to rotate relative to the first transmission part 510 during the rotation of the handle component 300. In this way, problems, such as wire twining and a limited rotation range, which are caused by the rotation of the handle component 300, can be avoided.

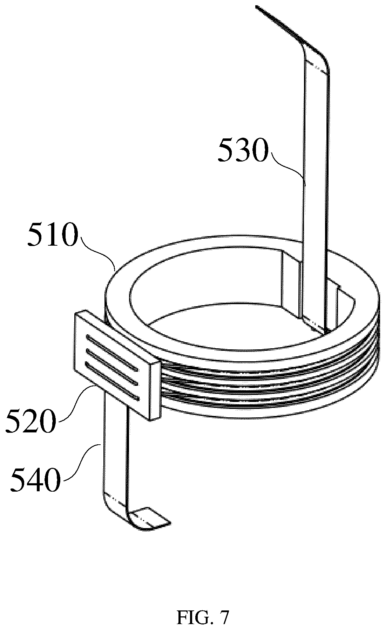

When the signal transmission component 500 includes a first transmission part 510 and a second transmission part 520, referring to FIG. 7, in some embodiments of this disclosure, the first transmission part 510 includes a first conductor, and the second transmission part 520 includes a second conductor. The first conductor and the second conductor are in contact with each other, and at least one of the first conductor and the second conductor is a circular structure. In this way, the first transmission part 510 can remain relatively stationary, the first conductor and the second conductor can remain conductive at all times. It can be understood that, with the above structure, both the first transmission part 510 and the key component 200 are stationary, and there is no relative movement between them. The second transmission part 520 moves synchronously with the handle component, and there is no relative movement between them. The first transmission part 510 and the second transmission part 520 are conducted with each other through contact, and there are no wires between them. Therefore, the problem of wire twining can be completely solved. At the same time, the handle component can rotate freely, without the limitation of a rotation range.

Taking FIG. 7 as an example, the first conductor is a circular structure, and the second conductor is a slider. The second conductor can rotate along a circumference of the first conductor on an outer side of the first conductor. As a specific way of contact conduction between the first conductor and the second conductor, an outer surface of the first conductor is provided with at least one circular conductive groove, and the corresponding conductive groove of the second conductor is provided with a conductive needle. The conductive needle is located inside the conductive groove and contacted with a groove wall for conduction. In other specific ways, the first conductor is a slider, the second conductor is a circular structure, or both the first conductor and the second conductor are circular structures, and the two circular structures are interlocked with each other.

When the signal transmission component 500 includes a first transmission part 510 and a second transmission part 520, a signal which is transmitted between the first transmission part 510 and the second transmission part 520 in some embodiments of this disclosure is an electrical signal. For example, when the first transmission part 510 and the second transmission part 520 are respectively the aforementioned first conductor and second conductor, they are contacted with each other to be conducted, so as to transmit the electrical signal.

When the signal transmission component 500 includes the first transmission part 510 and the second transmission part 520, other signals can also be transmitted for control. The first transmission part 510 and the second transmission part 520 can achieve signal transmission without contact. In another embodiment of this disclosure, a signal which is transmitted between the first transmission part 510 and the second transmission part 520 is a magnetic field signal. Correspondingly, the first transmission part 510 includes an element for emitting magnetic field, and the second transmission part 520 includes an element for receiving magnetic field. In response to a triggering of the key component 200, the element for emitting magnetic field emits a magnetic field signal to the element for receiving magnetic field.

In another embodiment of this disclosure, a signal which is transmitted between the first transmission part 510 and the second transmission part 520 is an optical signal. Correspondingly, the first transmission part 510 includes an element for emitting light, and the second transmission part 520 includes an element for receiving light. In response to a triggering of the key component 200, the element for emitting light emits an optical signal to the element for receiving light.

In another embodiment of this disclosure, a signal which is transmitted between the first transmission part 510 and the second transmission part 520 is an electromagnetic wave signal. Correspondingly, the first transmission part 510 includes an element for emitting electromagnetic wave, and the second transmission part 520 includes an element for receiving electromagnetic wave. In response to a triggering of the key component 200, the element for emitting electromagnetic wave emits an electromagnetic wave signal to the element for receiving electromagnetic wave. For example, the first transmission part 510 is a wireless transmission module, and the second transmission part 520 is a wireless receiving module.

In another embodiment of this disclosure, a signal which is transmitted between the first transmission part 510 and the second transmission part 520 is a sound signal. Correspondingly, the first transmission part 510 includes an element for emitting sound, and the second transmission part 520 includes an element for receiving sound. In response to a triggering of the key component 200, the element for emitting sound emits a sound signal to the element for receiving sound. For example, the element for emitting sound can emit a sound wave of a specific wavelength (or frequency), and/or emit a sound wave according to a specific rule (such as a duration, frequency, etc.), so that the element for receiving sound can recognize them.

When the signal transmission component 500 includes a first transmission part 510 and a second transmission part 520, referring to FIGS. 4 and 7, in some embodiments of this disclosure, the signal transmission component 500 further includes a first flexible circuit board 530. The first transmission part 510 is electrically connected with the key component 200 through the first flexible circuit board 530. It can be understood that since both the first transmission part 510 and the key component 200 remain stationary, the first flexible circuit board 530 is not twisted. In this embodiment, the first flexible circuit board 530 with a customizable length is configured to connect the first transmission part 510 and the key component 200. The installation positions of the first transmission part 510 and the key component 200 are not limited by the first transmission part 510, so that the key component 200 can be arranged at a position that can be avoided from being accidentally triggered by the user as much as possible. For example, the key component 200 can be arranged at the first section 111, which is far away from the second end 1112 of the handle component 300.

When the signal transmission component 500 includes a first transmission part 510 and a second transmission part 520, referring to FIGS. 4 and 7, in some embodiments of this disclosure, the signal transmission component 500 also includes a second flexible circuit board 540. The second transmission part 520 is electrically connected with the image processing component 320 through the second flexible circuit board 540. It can be understood that since the second transmission part 520 and the image processing component 320 can move synchronously and remain relatively stationary, the second flexible circuit board 540 therebetween is not twisted due to the rotation of the handle component 300. In this embodiment, the second transmission part 520 is connected with the image processing component 320 through the second flexible circuit board 540 with a customizable length, and the installation positions of the second transmission part 520 and the image processing component 320 are not limited by the second flexible circuit board 540.

Based on the first embodiment and referring to FIGS. 4 to 6, in some embodiments of this disclosure, one end (e.g., the right end in FIG. 4) of the second section 112 of the first connection part 110 is connected with the first section 111, and the other end (e.g., the left end in FIG. 4) of the second section 112 of the first connection part 110 is connected with the second connection part 120. A first end 1111 (e.g., the lower end in FIG. 4) of the first section 111, which end is away from the second section 112, is connected with the handle component 300, so as to form an L-shaped structure.

In this embodiment, the key component 200 is arranged at a second end 1112 (such as the upper end in FIG. 4) of the first section 111, which end is away from the handle component 300. Since the second end 1112 is away from the handle component 300, it can further reduce the probability of accidental triggering of the key component 200 when the user rotates the handle component 300.

It should be noted that when this disclosure describes one member is connected with one end of another member, it roughly defines the positional relationship between the two members, that is, the one member is closer to said one end of said another member, rather than means that the one member must be connected with a top end of said another member.

Based on the first embodiment, in another embodiment of this disclosure, the key component 200 can also be arranged at the second section 112 of the first connection part 110, which further increases a distance between the key component 200 and the handle component 300, so as to reduce the probability of accidental triggering.

When the key component 200 is arranged at the second end 1112 of the first section 111, which end is away from the handle component 300, referring to FIG. 3, in some embodiments of this disclosure, the key component 200 is arranged at a side of the first section 111, which side faces away from the second section 112 (that is, a side which faces the user), and is inclined towards the second section 112, so as to facilitate the user to trigger the key component 200 through the thumb when holding the handle component 300. Taking FIG. 3 as an example, in order to accommodate the inclined installation of the key component 200, the side of the first section 111, which side faces away the second section 112, has a slope or a curved surface, and the key component 200 is installed on the slope or the curved surface.

Based on the first embodiment, in another embodiment of this disclosure, the handle component 300 is configured to be driven by an external force to rotate relative to the key component 200, so as to facilitate the user to operate the handle component 300 to return to the correct position.

A second embodiment of this disclosure also provides an imaging apparatus for a rigid endoscope, including a connection component 100, a key component 200, a handle component 300, and an optical component 400. The connection component 100 is configured to install an external endoscope; the handle component 300 is configured to be held by a user and/or used by a user to implement a manual adjustment; the key component 200 is capable of being triggered by the user to control the imaging apparatus 1000 to perform an operation; the optical component 400 is configured to form an optical path. The connection component 100, the key component 200, the handle component 300, and the optical component 400 can all be understood with reference to the aforementioned embodiments.

The connection component 100 in this embodiment includes a first connection part 110 and a second connection part 120 that are interconnected with each other. The first connection part 110 is connected with the handle component 300, and the second connection part 120 is connectable with an external endoscope. Specifically, the first connection part 110 includes a first section 111 and a second section 112 that are interconnected with each other, for example, the first section 111 and the second section 112 are connected with each other as an integrated structure. The first section 111 is connected with the handle component 300, and the second section 112 is connected with the second connection part 120. In the illustrated embodiment, the first section 111 and the second section 112 are connected with each other through their respective ends.

One end (e.g. the right end in FIG. 4) of the second section 112 of the first connection part 110 is connected with the first section 111, and the other end (e.g. the left end in FIG. 4) of the second section 112 of the first connection part 110 is connected with the second connection part 120. A first end 1111 (e.g. the lower end in FIG. 4) of the first section 111, which end is away from the second section 112, is connected with the handle component 300. The first section 111 is provided inside with a first channel 113, and the second section 112 is provided inside with a second channel 114. The first channel 113 and the second channel 114 are jointed with each other. Taking the example shown in the figure, the first channel 113 and the second channel 114 are jointed with each other through their respective ends. More specifically, the first channel 113 is arranged substantially along a vertical direction, and the second channel 114 is arranged substantially along a horizontal direction, so that the first channel 113 and the second channel 114 are substantially perpendicular to each other. The optical component 400 includes a first optical element 410 arranged inside the first channel 113, and a second optical element 420 arranged inside the second channel 114. An optical axis of the first optical element 410 is substantially perpendicular to an optical axis of the second optical element 420.

In this embodiment, the key component 200 is arranged at a second end 1112 of the first section 111, which end is away from the handle component 300 (such as the upper end in FIG. 4). Since the second end 1112 is away from the handle component 300, it can further reduce the probability of accidental triggering of the key component 200 when the user rotates the handle component 300.

Based on the second embodiment, referring to FIGS. 4 to 6, in some embodiments of this disclosure, one end of the second section 112 is connected with the second end 1112 of the first section 111 to form an L-shaped structure. It should be noted that the "L-shaped structure" referred to in this disclosure should be understood as the first connection part 110 has a turn to form two sections, and should not be limited to that the first section 111 and the second section 112 are strictly perpendicular to each other.

Based on the second embodiment, in some embodiments of this disclosure, a central axis of the first channel 113 is substantially perpendicular to a central axis of the second channel 114, thereby facilitating installations of the first optical element 410 and of the second optical element 420 with perpendicular optical axes inside.

Based on the second embodiment, referring to FIG. 3, in some embodiments of this disclosure, the key component 200 is arranged at a side of the first section 111, which side faces away from the second section 112, that is, the side which faces the user, so as to facilitate the user to trigger the key component 200 through the thumb when holding the handle component 300. In addition, the key component 200 can be further inclined towards the second section 112, as shown in FIG. 3, so as to facilitate the user to trigger the key component 200. For example, the side of the first section 111, which side faces away from the second section 112, has a slope or a curved surface, and the key component 200 is installed on the slope or the curved surface.

The embodiments of this disclosure have been described in detail above in conjunction with the accompanying drawings, but this disclosure is not limited to the above embodiments. Various changes can be made within the knowledge scope of one skilled in the relevant technical field without departing from the purpose of this disclosure. In addition, the embodiments and features of this disclosure can be combined with each other without conflict.

## Claims

1. An imaging apparatus for a rigid endoscope, **characterized in that**, comprising: a connection component, a key component, a handle component, and an optical component; wherein:
the key component is capable of being triggered by a user;
the handle component comprises a housing and an image processing component; wherein the image processing component is accommodated inside an inner cavity of the housing, and is configured to receive an optical signal which is transmitted by the optical component, and generate an image signal;
the connection component comprises a first connection part and a second connection part, wherein the second connection part is capable of being connected with the rigid endoscope, which connection is capable of being disconnected; the first connection part comprises a first section and a second section, wherein the first section is connected with the handle component, the second section is connected with the second connection part; the first section is provided with a first channel and the second section is provided with a second channel, wherein the first channel and the second channel are joined together; the optical component comprises a first optical element which is arranged inside the first channel, and a second optical element which is arranged inside the second channel; wherein an optical axis of the first optical element is substantially perpendicular to an optical axis of the second optical element;
wherein the key component is arranged at the first connection part, and electrically connected with the image processing component; the handle component is capable of being driven by an external force to move relative to the key component; the image processing component is further configured to receive the light signal and generate the image signal, in response to a triggering of the key component.

2. The imaging apparatus for a rigid endoscope according to claim 1, **characterized in that**, further comprising a signal transmission component, which comprises a first transmission part and a second transmission part; wherein the first transmission part is connected with the first connection part, and is further electrically connected with the key component; the second transmission part is connected with the handle component, and is further electrically connected with the image processing component; wherein the second transmission part is capable of rotating relative to the first transmission part, during a rotation of the handle component.

3. The imaging apparatus for a rigid endoscope according to claim 2, **characterized in that**, the first transmission part comprises a first conductor, the second transmission part comprises a second conductor; wherein the first conductor and the second conductor are in contact with each other, and at least one of the first conductor and the second conductor is a circular structure.

4. The imaging apparatus for a rigid endoscope according to claim 2, **characterized in that**, a signal, which is transmitted between the first transmission part and the second transmission part, is an electrical signal.

5. The imaging apparatus for a rigid endoscope according to claim 2, **characterized in that**, a signal, which is transmitted between the first transmission part and the second transmission part, comprises at least one of: a magnetic field signal, an optical signal, an electromagnetic wave signal, and a sound signal.

6. The imaging apparatus for a rigid endoscope according to claim 5, **characterized in that**:
the first transmission part comprises an element for emitting magnetic field, the second transmission part comprises an element for receiving magnetic field; the element for emitting magnetic field is configured to emit a magnetic field signal to the element for receiving magnetic field, in response to the triggering of the key component;
the first transmission part comprises an element for emitting light, the second transmission part comprises an element for receiving light; the element for emitting light is configured to emit an optical signal to the element for receiving light, in response to the triggering of the key component;
the first transmission part comprises an element for emitting electromagnetic wave, the second transmission part comprises an element for receiving electromagnetic wave; the element for emitting electromagnetic wave is configured to emit an electromagnetic wave signal to the element for receiving electromagnetic wave, in response to the triggering of the key component;
the first transmission part comprises an element for emitting sound, the second transmission part comprises an element for receiving sound; the element for emitting sound is configured to emit a sound signal to the element for receiving sound, in response to the triggering of the key component.

7. The imaging apparatus for a rigid endoscope according to claim 2, **characterized in that**, the signal transmission component further comprises a first flexible circuit board, wherein the first transmission part is further electrically connected with the key component through the first flexible circuit board; and/or
the signal transmission component further comprises a second flexible circuit board, wherein the second transmission part is further electrically connected with the image processing component through the second flexible circuit board.

8. The imaging apparatus for a rigid endoscope according to claim 1, **characterized in that**, one end of the second section of the first connection part is connected with the first section of the first connection part, the other end of the second section is connected with the second connection part; wherein a first end of the first section, which first end is away from the second section, is connected with the handle component;
wherein, the key component is arranged at a second end of the first section, which second end is away from the handle component.

9. The imaging apparatus for a rigid endoscope according to claim 8, **characterized in that**, the key component is arranged at a side of the first section, which side faces away the second section.

10. The imaging apparatus for a rigid endoscope according to claim 1, **characterized in that**, the handle component is configured to be driven by an external force to rotate relative to the key component.

11. An imaging apparatus for a rigid endoscope, **characterized in that**, comprising: a connection component, a key component, a handle component, and an optical component; wherein:
the key component is capable of being triggered by a user;
the handle component is configured to be held by a user and/or used by a user to implement a manual adjustment;
the connection component comprises a first connection part and a second connection part, wherein the second connection part is capable of being connected with the rigid endoscope, which connection is capable of being disconnected; the first connection part comprises a first section and a second section; one end of the second section is connected with the first section, the other end of the second section is connected with the second connection part; a first end of the first section, which first end is away from the second section, is connected with the handle component; the first section is provided with a first channel and the second section is provided with a second channel, wherein the first channel and the second channel are joined together; the optical component comprises a first optical element which is arranged inside the first channel, and a second optical element which is arranged inside the second channel; wherein an optical axis of the first optical element is substantially perpendicular to an optical axis of the second optical element;
wherein, the key component is arranged at a second end of the first section, which second end is away from the handle component.

12. The imaging apparatus for a rigid endoscope according to claim 11, **characterized in that**, the one end of the second section is connected with the second end of the first section, so as to form an L-shaped structure.

13. The imaging apparatus for a rigid endoscope according to claim 11, **characterized in that**, a central axis of the first channel is substantially perpendicular to a central axis of the second channel.

14. The imaging apparatus for a rigid endoscope according to claim 11, **characterized in that**, the key component is arranged at a side of the second end, which side faces away the second section.

15. An endoscope system, **characterized in that**, comprising:
a light source host;
an imaging host;
a light guide beam, wherein one end of the light guide beam is connected with the light source host, the other end of the light guide beam is connected with a rigid endoscope, wherein the light guide beam is configured to provide a light source for the external endoscope; and
the imaging apparatus according to any one of claims 1-14, wherein the imaging apparatus is connected with the imaging host through a communication cable, so as to transmit an image signal which is obtained by the imaging apparatus to the imaging host.
